# EUROPÄISCHE PATENTANMELDUNG

(11) **EP 0 686 845 A2**
(43) Veröffentlichungstag der Anmeldung: **13.12.1995**
(21) Anmeldenummer: 95108017.5
(22) Anmeldetag: 24.05.1995
(51) Int. Cl.: G01N 25/72

(54) **Prooftest für keramische Bauteile**

(30) Priorität: 06.06.1994 DE 4419750
(71) Anmelder: SIEMENS AKTIENGESELLSCHAFT, D-80333 München (DE)
(72) Erfinder: Bast, Ulrich, Dr., D-81667 München (DE); Kempter, Karl, Dr., D-81477 München (DE); Schulenberg, Thomas, Dr., D-45219 Essen (DE)

(57) **Zusammenfassung**

Zum Testen strukturkeramischer Bauteile unter Überlastbedingungen wird vorgeschlagen, auf dem Bauteil durch Wärmestrahlungseinwirkung eine vorher errechnete Temperaturverteilung zu erzeugen, welche die gewünschte Prüfspannung bzw. Überlast induziert. Mittels orts- und zeitauflösender Temperatursensoren wird die Temperaturverteilung gemessen, mit einer gewünschten bzw. berechneten Temperaturverteilung verglichen und durch Veränderung von Dauer und/oder Ort der Strahleneinwirkung geregelt, bis die gewünschte Temperaturverteilung erzeugt ist.

## Beschreibung

Die von keramischen Werkstoffen wie zum Beispiel Siliziumnitrid oder Siliziumkarbid erreichte hohe Festigkeit erlaubt den Einsatz dieser Materialien für Strukturanwendungen, beispielsweise für Hitzeschilde oder Turbinenschaufeln. Im Gegensatz zu metallischen Werkstoffen behalten die Keramiken ihre hohe Festigkeit bis hin zu Temperaturen weit oberhalb von 1000°C. Dies und die hohe Korrosionsbeständigkeit eröffnen für die Keramik verschiedene Anwendungen als Konstruktionswerkstoff, insbesondere im Hochtemperaturbereich.

Durch die Verwendung von Keramik lassen sich technische Konzepte realisieren, die bisher am Fehlen geeigneter Werkstoffe scheiterten. Diese Konzepte haben meist geringere Agasemissionen und Einsparung von Brennstoff zum Ziel. Ein Beispiel dafür ist das Erreichen höherer Wirkungsgrade in Fahrzeugund Stationärgasturbinen durch die Steigerung der Arbeitstemperatur. Ein weiteres Beispiel ist eine verlustärmerer und ruhigerer Antrieb bei keramischen Ein- und Auslaßventilen von Kolbenmotoren durch die Verringerung dem Reibung und der oszillierenden Massen.

Beim Einsatz von keramischen Bauteilen können jedoch auch Probleme auftreten, die durch Keramik-typische Nachteile wie Sprödigkeit und Streuung der mechanischen Eigenschaften bedingt sind. Keramiken können herstellungsbedingte Fehler wie Mikrorisse, Poren, Agglomerate usw. aufweisen. Diese können unter thermisch und mechanisch belastenden Betriebsbedingungen zu einer lokalen Spannungsüberhöhung führen, die in der Keramik nicht durch plastische Verformung, sondern nur durch Rißbildung und -wachstum abgebaut werden können. Wird die kritische Belastung überschritten, führt dies zum Ausfall des Bauteils.

Da diese für Keramiken typischen Strukturfehler bezüglich ihrer Häufigkeit, Form und Größe in statistischer Verteilung auftreten, kann die Zuverlässigkeit keramischer Bauteile nur gesteigert werden, wenn alle Bauteile, die Fehler oberhalb einer bestimmten Fehlergröße enthalten, vor ihrem bestimmungsgemäßen Einsatz bzw. vor dem Einbau durch einen Prooftest ausgesondert werden. Bei einem Prooftest werden alle Bauteile mit einer Prüfspannung belastet, die größer ist, als die maximale im Betrieb auftretende Spannung. Die Höhe der Prüfspannung hängt von der maximalen Einsatzbelastung und der angestrebten Lebensdauer ab. Letztere wiederum ist ebenfalls von der Anfangsrißlänge abhängig, aus der sich bei bekanntem unter-kritischem Rißwachstum die Zeit bis zum Erreichen einer kritischen Rißgröße errechnen läßt.

Beim Prooftest sollen die im Betrieb auftretenden Belastungsbedingungen möglichst genau simuliert werden. Besonders bei Bauteilen, bei denen die Betriebsbelastungen hauptsächlich durch Temperaturgradienten hervorgerufen werden, kann die Spannungsverteilung in der Regel aber nicht durch rein mechanisches Testen erzeugt werden, ohne daß an wahrend des Betriebs geringer belasteten Stellen stark überhöhte Spannungen entstehen. Dies hätte eine zu große Anzahl von Brüchen während des Überlasttests zur Folge.

Unterschiedliche Anwendungen keramischer Bauteile können unterschiedliche Bauteilgeometrien und unterschiedliche Belastungszustände erzeugen. Daher gibt es kein Standardverfahren für einen Überlasttest. Für Einzelanwendungen existieren bereits spezifische Überlasttests. Beispielsweise können Keramikkugeln für Hüftgelenksprothesen durch das definierte Einpressen eines Konus in die Aufnahmebohrung für den Schaft geprüft werden.

Turboladerrotoren und Schaufelräder von Fahrzeuggasturbinen können vor dem Einbau im kalten Zustand einem Test mit Überdrehzahl unterzogen werden. Auch keramische Schleifscheiben werden auf diese Weise geprüft. Nachteilig an diesem Verfahren ist, daß Warmespannungen und Vibrationen nicht berücksichtigt werden und daß es auf rotationssymmetrische Teile beschränkt ist.

Mit Hilfe des Thermoschockverfahrens wird beispielsweise versucht, die durch Wärmespannungen induzierten Belastungen während des Betriebs zu simulieren. Dabei werden die Bauteile durch Gasbrenner erhitzt. Die so erzeugten Spannungen werden aus der gemessenen Temperaturverteilung errechnet. Mit diesem Verfahren konnte allerdings die Ausfallwahrscheinlichkeit keramischer Bauteile wahrend deren Betrieb nur geringfügig verbessert werden. Vermutlich liegt dies daran, daß bei der Erhitzung durch den Brenner die Temperaturverteilung nicht steuerbar ist. In deren Folge stimmt auch die so erzeugte Spannung nicht mit den Verhältnissen beim bestimmungsgemäßen Betrieb des Bauteils überein. Auch ist es möglich, daß während des Prüftests nicht alle Teile des zu prüfenden Bauteils einer die übliche Betriebsspannung überschreitenden Prüfspannung ausgesetzt werden.

Aufgabe der vorliegenden Erfindung ist es daher, ein Verfahren zum Testen keramischer Bauteile anzugeben, mit dem sich betriebsspezifische Prüfspannungen in dem keramischen Bauteil erzeugen lassen, welches einfach durchzuführen ist und mit dem sich schadhafte Bauteile ebenso sicher aussortieren lassen wie solche Bauteile, die über einem Grenzwert liegende Rißlängen aufweisen.

Diese Aufgabe wird erfindungsgemäß gelöst mit einem Verfahren nach Anspruch 1. Weitere Ausgestaltungen der Erfindung sowie eine bevorzugte Verwendung für das Verfahren sind den Unteransprüchen zu entnehmen.

Mit dem erfindungsgemäßen Verfahren ist es möglich, durch Strahlungseinwirkung exakt die Temperaturverteilung auf dem Bauteil zu erzeugen, die die gewünschte Prüfspannung erzeugt.

Dies wird einerseits durch die Art der Energieübertragung (durch Einwirkung von Wärmestrahlung) und andererseits durch die erfindungsgemäß vorgeschlagene Regelung erreicht.

Durch Strahlungseinwirkung läßt sich in kürzester Zeit ausreichend Energie in das keramische Bauteil einkoppeln, um die gewünschten Temperaturen zu erzeugen, die bei über 1000°C liegen können. Andererseits läßt sich die Strahlung beliebig bündeln, so daß die Energie auf kleinstem Raum mit hoher Ortsgenauigkeit eingekoppelt werden kann. Die Energieeinkopplung ist dabei deutlich schneller als die Wärmeleitung, die zur Temperaturangleichung und damit zur Verfälschung der gewünschten Temperaturverteilung führt.

Zur Regelung des Verfahrens sind ortsauflösende Temperatursensoren vorgesehen, die die Temperatur des Bauteils an mehreren spezifischen Punkten möglichst genau bestimmen. Die so gemessene Temperaturverteilung wird mit einer gewünschten berechneten Temperaturverteilung verglichen und durch entsprechende Beeinflussung der Strahlungsquelle an die gewünschte Temperaturverteilung angeglichen. Vergleich und Regelung der Temperaturverteilung kann in einem einfachen Regelkreis erfolgen. Möglich ist es auch, dies über einen Rechner zu steuern.

Je nach Größe des zu testenden keramischen Bauteils und der damit verbundenen Wärmekapazität kann außerdem ein schneller Temperatursensor erforderlich sein, um eine genaue Temperaturregelung zu ermöglichen.

Als Strahlungsquellen sind insbesondere Laser geeignet. Möglich ist es jedoch auch, außerdem Halogenlampen, Bogenlampen und ähnliche einzeln oder in Kombination zu verwenden.

Die gewünschte Temperaturverteilung erfolgt in definierter Weise durch die zeitliche und örtliche Steuerung der Strahlungseinwirkung. Die zeitliche Steuerung kann neben der Dauer der Einwirkung außerdem eine Beeinflussung der Pulsfrequenz der Strahlung beinhalten.

Da sowohl örtliche und zeitliche Einwirkung der Strahlung genau eingestellt werden können, ist das Verfahren an in weiten Grenzen variierbare Bauteilgeometrien anpaßbar.

Extreme Temperaturverteilungen bzw. Temperaturverteilungen mit einem hohen Temperaturgradienten können außerdem durch aktive Kühlung des Bauelements an geeigneten Orten unterstützt werden.

Eine örtliche Veränderung der Strahlungseinwirkung kann bei Verwendung eines Lasers beispielsweise durch Ablenkung des Laserstrahls mit Hilfe zweier computergesteuerter Schwingspiegel vorgenommen werden. Die Achsen der Schwingspiegel sollten dabei senkrecht zueinander stehen, so daß auch kompliziert geformte heiße Zonen mit in weiten Grenzen wählbaren Temperaturgradienten im zu prüfenden Bauteil erzeugt werden können. Größere örtliche Auslenkungen der Strahlungseinwirkung werden durch Relativbewegung zwischen Strahlenquelle und Bauteil erzeugt. Beispielsweise kann dazu der Laser fest angeordnet werden, während das Bauteil auf einem Meßtisch befestigt wird, welcher beispielsweise entlang zweier senkrecht zueinander stehender Achsen mit Hilfe von Schrittmotoren bewegt werden kann.

Bei Bauteilen mit einfacher Geometrie und insbesondere bei kleinen zu testenden Bauteilen ist es auch möglich, diese mit einer geeigneten Transportvorrichtung kontinuierlich oder quasi kontinuierlich an der Strahlungsquelle vorbeizuführen. Auf diese Weise können beispielsweise auch funktionskeramische Bauteile getestet werden.

Ein strukturkeramisches Bauteil kann während seines bestimmungsgemäßen Gebrauchs mehreren unterschiedlichen Spannungsfeldern ausgesetzt sein. Im erfindungsgemäßen Prooftest werden solche unterschiedlichen Spannungsfelder durch unterschiedliche Temperaturverteilungen simuliert. Nach dem Erzeugen einer ersten Temperaturverteilung im Bauelement wird dieses wieder abgekühlt und anschließend durch erneute Strahlungseinwirkung an anderem Ort oder mit anderem Temperaturgradienten eine andere Temperaturverteilung und damit eine andere Prüfspannung erzeugt.

Der Verlauf einer gewünschten Temperaturverteilung bzw. die Lage des Gradienten der Temperaturverteilung kann beispielsweise auf einer Linie liegen, entlang der die größten Kräfte während des Betriebs des Bauteils auftreten. Ein solcher Gradient läßt sich durch Strahlungseinwirkung auf einem eng begrenzten Bereich des Bauteils erzeugen, beispielsweise durch punktförmige Strahlungseinwirkung. Der Ort der Strahlungseinwirkung wird auch in Anhängigkeit von der Bauteilgeometrie gewählt und kann beispielsweise in der Nähe einer stabilitätsbedingten Schwachstelle des Bauelements liegen. Möglich ist es auch, die Strahlungseinwirkung über einen größeren Bereich des Bauteils zu verteilen. Bei rotationssymmetrisch geformten Bauteilen ist es beispielsweise möglich, eine ringförmige erhitzte Zone zu erzeugen, wobei eine radiale Temperaturverteilung bzw. ein radial zum Rotationsmittelpunkt hin abfallender Temperaturgradient erzeugt wird.

Im folgenden wird die Erfindung anhand eines Ausführungsbeispiels und der dazugehörigen zwei Figuren näher erläutert.

Figur 1 zeigt eine Anordnung zur Durchführung des Verfahrens.

Figur 2 zeigt das Verfahren als Blockschaltbild.

### Ausführungsbeispiel:

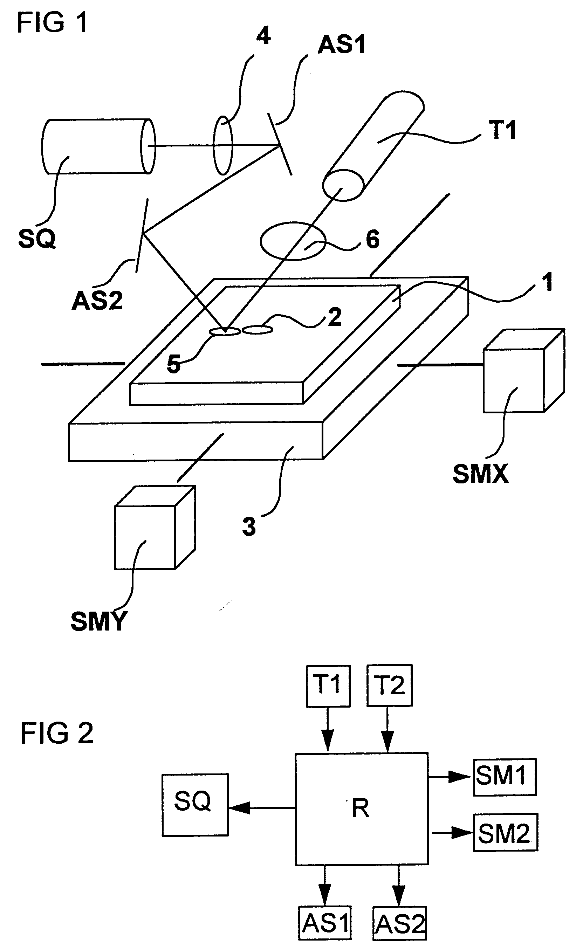

Figur 1: Im erfindungsgemäßen Verfahren soll die Überlastprüfung eines keramischen Hitzeschildes durchgeführt werden. Als Modell für einen Hitzeschild dient eine quadratische Platte 1 mit 200 mm Kantenlänge und einer Dicke von 5 mm. Die Platte 1 ist beispielsweise aus Siliziumnitrid oder Siliziumkarbid ausgeführt. In der Mitte der Platte 1 befindet sich für Befestigungszwecke eine Bohrung 2 mit einem Durchmesser von 20 mm.

Die Platte wird auf einem Meßtisch 3 angeordnet. Zwei Schrittmotoren SMX und SMY ermöglichen eine Translation des Meßtisches entlang der X- und der Y-Achse.

Als Strahlungsquelle SQ dient ein Laser, beispielsweise ein Nd:YAG-Laser. Dieser ist aufgrund seiner Wellenlänge von ca. 1,06 µm insbesondere für nichtoxidische Keramik geeignet und erbringt die erforderliche hohe Strahlungsleistung, mit der eine schnelle Energieeinkopplung möglich ist. Der weitere Vorteil des Festsstofflasers ist die durch seine Wellenlänge eröffnete Möglichkeit, die zur Strahlungsbündelung und Strahlenführung dienende Optik 4 aus Glas auszuführen.

Zur Ablenkung des Laserstrahls dient ein erster und ein zweiter Ablenkspiegel AS1 bzw. AS2, die jeweils um eine Achse drehbar sind, wobei die beiden Achsen vorzugsweise senkrecht zueinander stehen. Mit Hilfe der Optik 4 und der Ablenkspiegel AS wird der vom Laser SQ erzeugte Strahl auf das Bauteil 1 gerichtet, wo er einen heißen Fleck (Spot) von ca. 10 mm Durchmesser erzeugt. Da die bezüglich der mechanischen Stabilität des Bauteils 1 kritischste Stelle nahe der Bohrung 2 liegt, wird auch der Spot in einem Abstand von ca. 5 mm zur Bohrung 2 auf die Platte 1 gerichtet.

Zur Kontrolle der Temperaturverteilung, die im vorliegenden Fall zumindest einen radial zur Bohrung 2 gerichteten Temperaturgradienten umfaßt, sind zumindest zwei Temperatursensoren T1 und T2 erforderlich, von denen in der Figur der Übersichtlichkeit halber nur einer dargestellt ist. Für genauere Messungen der Temperaturverteilung können weitere Temperatursensoren erforderlich sein.

Als Temperatursensor T1 kann ein den Photoeffekt ausnützendes Halbleiterbauelement verwendet werden, beispielsweise eine im infraroten empfindlichen Photodiode. Mit dem genannten Sensor läßt sich die Temperatur in einfacher Weise auf eine Genauigkeit von ± 5^{o}C messen. Für das im Ausführungsbeispiel verwendete Bauteil 1 ist eine Zeitauflösung des Temperatursensors von 10 msec ausreichend. Bei kleineren Bauteilen mit geringerer Wärmekapazität ist eine schnellere Bestimmung der Temperaturverteilung erforderlich, so daß eine Einzelmessung eines Temperatursensors T beispielsweise innerhalb 800 µsec erfolgen muß. Die Ortsgenauigkeit der Temperaturmessung ist so ausgelegt, daß beispielsweise ein Temperaturgradient von 300 K/mm bestimmt werden kann. Da die Temperaturmessung auf "optischem" Weg erfolgt, kann sie durch eine geeignete Optik 6 verbessert werden. Das Meßverfahren erlaubt auch einen ausreichenden Meßabstand des Temperatursensors T zum Bauteil 1, welcher beispielsweise 15 bis 23 cm betragen kann. Mit geeignet gewählten Temperatursensoren sollten bei gleicher Meßgenauigkeit auch größere Meßabstände möglich sein, die vorteilhaft für das Verfahren sind.

Figur 2 erläutert die Durchführung des Verfahrens anhand eines schematischen Blockschaltbilds. Eine für das Verfahren erforderliche bzw. gewünschte Prüfspannung wird nach der Finite-Elementmethode berechnet. Unter Berücksichtigung der Materialeigenschaften und der im Betrieb auftretenden Bedingungen, welche beispielsweise mechanische Belastungen oder vorzugsweise thermisch induzierte Spannungen sind, wird eine diese Prüfspannung erzeugende Temperaturverteilung für das Bauteil 1 berechnet. Die Kontrolle der Temperaturverteilung erfolgt anhand von für die spezielle Temperaturverteilung typischen Punkten.

Die an den Meßpunkten zu bestimmende Temperaturverteilung wird in einem zur Regelung des Verfahrens verwendeten Rechner R eingegeben. In einer vorgegebenen Meßfrequenz von beispielsweise 100 Hz werden die von den Temperatursensoren T1, T2 ... Tn ermittelten Temperaturwerte an den Rechner weitergeleitet und dort mit den vorgegebenen bzw. gewünschten Werten verglichen. Abweichungen von dem gewünschten Wert können durch Ausrichtung des Laserstrahls über die ebenfalls vom Rechner R gesteuerten Ablenkspiegel AS1 und AS2 erfolgen. Größere örtliche Abweichungen können auch durch die Schrittmotoren SMX und SMY nachgeregelt werden. Weiterhin kann die Temperaturverteilung über die Regulierung der Leistung der Strahlenquelle SQ erfolgen, wobei gegebenenfalls mehr oder weniger Leistung in den Spot 5 auf dem Bauteil 1 eingekoppelt wird.

Durch einen angekoppelten Ultraschallsensor werden die im Fall des Rißwachstums während des Anliegens der Prüflast entstehender Schallemissionen aufgenommen. Die Schallsignale dienen zum Nachweis des Bauteilversagens aufgrund der Prüfbelastung.

Ist die gewünschte Temperaturverteilung und die dadurch erzeugte Überlastspannung (Prüfspannung) erreicht, läßt man das Bauteil 1 abkühlen, gegebenenfalls durch aktive Kühlung.

Um allen Einsatzbedingungen des Bauteils 1 gërecht zu werden, wird das Verfahren nun wiederholt, wobei eine andere Temperaturverteilung und damit eine anders gerichtete Prüfspannung im Bauteil 1 erzeugt wird. Im gewählten Ausführungsbeispiel ist dabei eine weitere ortsfeste punktförmige Strahleneinwirkung auf einen Ort in der Nähe der Bohrung 5 geeignet.

Insgesamt werden durch mehrfache Wiederholung des Verfahrens 8 bis 10 heiße Spots auf dem Bauteil mit zwischenzeitlichem Abkühlen gemäß einer vorberechneten Prüfspannung bzw. Temperaturverteilung erzeugt. Bei der angegebenen Menge der Meßpunkte ist das Verfahren zum Beispiel bei Verwendung eines 800 Watt starken Festkörperlasers nach ca. 5 bis 15 Minuten beendet. Bei höherer Laserleistung reduziert sich der Zeitbedarf entsprechend. Nach der Durchführung des Verfahrens wird das Bauteil 1 optisch auf Beschädigung wie zum Beispiel Rißbildung ünberprüft und gegebenenfalls aussortiert. Unbeschädigte Bauteile enthalten nun nur noch Risse unter einer bestimmten Rißlänge, die durch die gewünschte Lebensdauer des Bauteils unter bestimmungsgemäßen Betriebsbedingungen bestimmt wird.

Neben dem beispielhaft angeführten Ausführungsbeispiel ist das erfindungsgemäße Testverfahren als Prooftest für beliebig geformte keramische Bauteile geeignet, wobei sich jeweils auch andere als die angeführten Temperturverteilungen auf dem Bauteil erzeugen lassen. Somit können beliebige Prüfspannungen simuliert werden.

## Patentansprüche

1. Verfahren zum Testen eines strukturkeramischen Bauteils (1) durch Ausbilden einer thermisch induzierten Prüfspannung, bei dem
- das Bauteil in vorgegebenen Bereichen (5) durch Strahlungseinwirkung erhitzt wird
- die Temperaturverteilung des Bauteils mittels ortsauflösender Temperatursensoren (T) ermittelt wird
- die gemessene Temperaturverteilung des Bauteils mit einer berechneten gewünschten Temperaturverteilung verglichen wird
- die Dauer und/oder der Ort der Strahlungseinwirkung geregelt wird, bis in dem Bauteil die gewünschte Temperaturverteilung erzeugt ist
- das Versagen des Bauteils durch Schallemissionsanalyse detektiert wird.

2. Verfahren nach Anspruch 1,
bei dem die Strahlungseinwirkung über einen Laser (SQ) erfolgt.

3. Verfahren nach Anspruch 1 oder 2,
bei dem zum Erzeugen der gewünschten Temperaturverteilung das Bauteil (1) zusätzlich aktiv gekühlt wird.

4. Verfahren nach einem der Ansprüche 1 bis 3,
bei dem die Temperaturverteilung mit in einem Abstand zum Bauteil (1) angeordneten Halbleitersensoren unter Ausnutzung des Photoeffekts ermittelt wird.

5. Verfahren nach einem der Ansprüche 1 bis 4,
bei dem das Bauteil (1) nach dem Erzeugen einer ersten Temperaturverteilung abgekühlt wird und bei dem anschließend in analoger Weise zumindest eine weitere von der ersten verschiedene Temperaturverteilung erzeugt wird.

6. Verfahren nach einem der Ansprüche 1 bis 5,
bei dem in dem Bauteil (1) über die Temperaturverteilung zumindest eine solch hohe Prüfspannung erzeugt wird, wie sie der bei der späteren bestimmungsgemäßen Verwendung des strukturkeramischen Bauteils auftretenden thermisch mechanischen Spannung entspricht.

7. Verfahren nach einem der Ansprüche 1 bis 6,
bei dem während der Strahlungseinwirkung eine Relativbewegung zwischen Bauteil (1) und Laser (SQ) durchgeführt wird.

8. Verfahren nach Anspruch 7,
bei dem die Relativbewegung über einen Schrittmotor (SMX, SMY) durchgeführt wird.

9. Verfahren nach Anspruch 7,
bei dem mehrere Bauteile (1) in geeigneter Ausrichtung an einer Strahlungsquelle (SQ) vorbei geführt werden.

10. Verwendung des Verfahrens als Prooftest für thermisch belastete keramische Bauteile wie Ventile, Hitzeschilde, Brennraumauskleidungen, Turbinenschaufeln oder funktionskeramische Bauelemente.
